# EUROPÄISCHE PATENTSCHRIFT

(11) **EP 3 126 461 B1**
(45) Veröffentlichungstag und Bekanntmachung des Hinweises auf die Patenterteilung: **24.10.2018**
(21) Anmeldenummer: 15713878.5
(22) Anmeldetag: 31.03.2015
(51) Int. Cl.: C09D 189/00, C09D 7/65, C09D 7/80, C07K 14/47, C08H 1/00, C09D 5/00, C12P 21/06, C12N 9/50, C12N 9/64, C12N 11/02, C12N 11/06, C12N 11/14

(54) **VERFAHREN ZUR BESCHICHTUNG VON OBERFLÄCHEN DURCH ENZYMATISCHE REAKTION**
METHOD FOR COATING SURFACES BY ENZYMATIC REACTION
PROCÉDÉ POUR LE REVÊTEMENT DE SURFACES PAR RÉACTION ENZYMATIQUE

(30) Priorität: 04.04.2014 DE 102014104859
(43) Veröffentlichungstag der Anmeldung: 08.02.2017
(73) Patentinhaber: Universität Paderborn, 33098 Paderborn (DE)
(72) Erfinder: STRUBE, Oliver Ingolf, 33102 Paderborn (DE); BREMSER, Wolfgang, 33100 Paderborn (DE); RÜDIGER, Arne Alexander, 33098 Paderborn (DE)
(74) Vertreter: Michalski Hüttermann & Partner Patentanwälte mbB
(86) Internationale Anmeldenummer: PCT/EP2015/056995
(87) Internationale Veröffentlichungsnummer: WO 2015/150368

(56) Entgegenhaltungen:
- EP-A1- 0 839 498
- WO-A1-96/07444
- WO-A1-99/59647
- US-A- 3 323 929
- US-A1- 2009 042 218
- US-A1- 2013 065 291

## Beschreibung

Die Erfindung betrifft das Gebiet der biologischen Beschichtungen. Insbesondere betrifft die Erfindung die Beschichtung von Oberflächen mit Biopolymeren über eine enzymatische Reaktion.

Biopolymere als Beschichtungsmaterialien weisen für viele Anwendungen vorteilhafte Eigenschaften auf, beispielsweise in Bezug auf Biokompatibilität, multifunktionale Oberflächen und biologische Abbaubarkeit. In den letzten Jahren wurde die Forschung im Bereich der Biopolymere stark intensiviert, von zentralem Interesse ist hierbei weiterhin das Aufbringen der Biopolymere auf feste Substratoberflächen. Biologische Beschichtungen werden bislang analog zu Polymerbeschichtungen mit klassischen Verfahren der Lackchemie hergestellt. Diese Beschichtungsverfahren wie Sprüh-, Tauch- oder Spin-Coating-Verfahren erlauben jedoch nur eine geringe Kontrolle über die Filmbildung oder ein Aufbringen komplexer Beschichtungen. Strukturen können lediglich im makroskopischen Bereich erzeugt werden, beispielsweise durch eine Maskierung. Dies bedingt jedoch ein Vielfaches an Verfahrensschritten und die Anwendung unterschiedlicher Verfahren zur Kontrolle der Filmeigenschaften. Es bedarf daher der weiteren Verbesserung der Herstellung von Beschichtungen mit Biopolymeren.

Die Schrift WO 2005/076987 offenbart die Möglichkeit einer Enzym-kontrollierten Beschichtung von Arzneimitteln, jedoch wird nicht offenbart wie eine derartige Beschichtung aufgebracht werden könnte.

Der vorliegenden Erfindung lag daher die Aufgabe zu Grunde, ein Verfahren zur Herstellung einer Beschichtung mit Biopolymeren unter Verwendung von Enzymen zur Verfügung zu stellen. Insbesondere war es die Aufgabe der vorliegenden Erfindung, ein Verfahren zur Verfügung zu stellen, das eine gezielte Beschichtung erlaubt.

Diese Aufgabe wird gelöst durch ein Verfahren zur Beschichtung von Oberflächen durch enzymatische Reaktion eines Biopolymers, wobei das Verfahren die folgenden Schritte umfasst:
a) Aufbringen eines Enzyms auf die Oberfläche eines Substrats, und
b) In-Kontakt-Bringen des Enzyms mit dem abzuscheidenden Biopolymer, wobei das Enzym das Biopolymer spaltet, wobei durch die Spaltung wenigstens zwei Spaltungsprodukte des Biopolymers mit unterschiedlicher Löslichkeit in einem Lösungsmittel entstehen, und wenigstens ein Spaltungsprodukt des Biopolymers mit geringerer Löslichkeit auf der Oberfläche des Substrats abgeschieden wird.

Erfindungsgemäß wird hierbei als Biopolymer das Protein Casein und als Enzym eine Protease ausgewählt aus der Gruppe umfassend Chymosin und/oder Pepsin verwendet.

Überraschend wurde gefunden, dass eine enzymatische Reaktion zur Auslösung eines Abscheidevorgangs genutzt werden kann. In vorteilhafter Weise kann die enzymatische Reaktion zur gezielten Kontrolle der Filmeigenschaften auf molekularer Ebene genutzt werden. So wird die Kontrolle von Filmeigenschaften wie Schichtdicke und Filmstruktur sowie die gezielte Beschichtung einzelner Bereiche eines Substrats ermöglicht. Durch das erfindungsgemäße Verfahren wird die präzise Beschichtung von Objekten komplexer Geometrie mit biologischen Filmen ermöglicht. In einem einzigen Prozess ist eine hohe Variabilität verschiedener Filmparameter realisierbar. Dies stellt eine immense Effizienzsteigerung von Beschichtungsverfahren und eine einfache Erreichbarkeit komplexer Filmeigenschaften zur Verfügung. In vorteilhafter Weise erlaubt die Abscheidung von Biopolymeren weiterhin die Herstellung biokompatibler Beschichtungen sowie biologisch abbaubarer Beschichtungen.

Der Gegenstand der vorliegenden Erfindung ist in den Ansprüchen 1-6 definiert. Die hierin beschriebenen Ausführungsformen, die nicht durch die Ansprüche abgedeckt sind, dienen lediglich der Veranschaulichung des technischen Kontextes der vorliegenden Erfindung.

Unter dem Begriff "Biopolymer" sind im Sinne der vorliegenden Erfindung Polymere biologischen Ursprungs zu verstehen. Von Lebewesen synthetisierte Polymere sind beispielsweise Polysaccharide und Proteine. So können Proteine als Polymere verstanden werden, die aus Aminosäuren-Monomeren ausgebildet sind.

Bevorzugte Biopolymere sind Proteine. Proteine können als isolierte Proteine vorliegen oder in Form eines Proteinkomplexes oder als Gemisch ähnlicher oder unterschiedlicher Proteine. Enzyme, die Biopolymer spalten können, sind insbesondere Proteasen. In bevorzugten Ausführungsformen ist das Biopolymer daher ein Protein, Proteinkomplex oder Proteingemisch und das Enzym eine Protease. Viele Polymer-Protease-Reaktionen sind bekannt und die entsprechenden Proteine und Proteasen kommerziell erhältlich oder in bekannter Weise isolierbar.

Durch eine Protease-induzierte Spaltung eines Biopolymers entstehen wenigstens zwei Spaltungsprodukte, wobei die Zahl der Spaltungsprodukte abhängig ist von der Anzahl der Stellen des Biopolymers, die die entsprechende Protease erkennt und spaltet. Die Spaltungsprodukte können je nach Art des Biopolymers unterschiedliche Eigenschaften aufweisen, beispielsweise eine unterschiedliche Löslichkeit in einem Lösungsmittel. Durch die Spaltung eines Proteins kann beispielsweise ein Molekülteil mit größerer Hydrophilie und damit erhöhter Löslichkeit in einem wässrigen Lösungsmittel und ein Molekülteil mit größerer Hydrophobizität und damit geringerer Löslichkeit in einem wässrigen Lösungsmittel entstehen. Wenn die Hydrophobizität eines Spaltungsprodukts des Proteins in einem wässrigen Lösungsmittel groß genug ist und sich dieses in der Nähe einer Oberfläche befindet, kann sich das hydrophobe Spaltungsprodukt bzw. das Spaltungsprodukt des Biopolymers mit geringerer Löslichkeit auf der Oberfläche des Substrats abscheiden. Bei dem In-Kontakt-Bringen des Enzyms mit dem abzuscheidenden Biopolymer liegt das Biopolymer vorzugsweise in Lösung vor. Ein bevorzugtes Lösungsmittel ist Wasser. Eine wässrige Lösung kann eine Lösung des Biopolymers in Wasser oder in einem wässrigen Gemisch sein.

Erfindungsgemäß wird das Protein Casein verwendet. Casein liegt in Milch oder wässrigen Lösungsmitteln in Form von Micellen vor, die hauptsächlich aus vier Casein-Proteinen, α_{S1}-Casein, α_{S2}-Casein, β-Casein und κ-Casein, ausgebildet werden. Casein kann somit auch als ein Gemisch von Proteinen betrachtet werden. Einfache Modelle der Caseinstruktur gehen davon aus, dass der Kern der Micelle hauptsächlich aus den hydrophoben α_{S1}-Casein, α_{S2}-Casein und β-Casein ausgebildet wird, während das amphiphile κ-Casein auf der äußeren Oberfläche der Micelle akkumuliert. Die Protease Chymosin spaltet κ-Casein an der Stelle der Phenylalanin¹⁰⁵-Methionin¹⁰⁶-Bindung. Hierdurch wird ein hydrophiler Teil (Caseinmakropeptid) abgespalten, während ein hydrophober, para-K-Casein genannter Teil, in der Micelle verbleibt. Dadurch resultiert insgesamt eine höhere Hydrophobizität der Micellen, die agglomerieren und beispielsweise aus Milch oder wässrigen Lösungsmitteln ausfallen können.

Erfindungsgemäß ist das Protein Casein und die Protease ist ausgewählt aus der Gruppe umfassend Chymosin und/oder Pepsin. Chymosin ist eine sogenannte Aspartat- oder Carboxylprotease, die unter Verbrauch eines Moleküls Wasser die Peptidbindungen eines Proteins spalten kann. Die enzymatische Spaltung von Casein durch Chymosin nutzt die komplexe Struktur der Caseinmicellen und die durch die Spaltung veränderte Löslichkeit der Micellen, um durch Abscheidung der nach der Spaltung hydrophoberen Micellen eine Beschichtung auszubilden. Ein weiterer Vorteil, der durch eine Beschichtung mit durch Chymosin gespaltenem Casein zur Verfügung gestellt wird, ist die höhere Hydrophobizität und damit höhere Beständigkeit der Caseinbeschichtung gegenüber Wasser verglichen mit Beschichtungen mit nicht gespaltenem Casein.

Die Spaltung des Biopolymers findet durch das erfindungsgemäß auf der Oberfläche eines Substrats aufgebrachte Enzym auf der Oberfläche des Substrats bzw. in dessen unmittelbarer Nähe statt. Die Größe bzw. Ausdehnung des Reaktionsbereichs, in dem das Enzym mit dem Biopolymer in Kontakt treten kann, auf bzw. über der Oberfläche kann durch die Methode der Immobilisierung des Enzyms an die Substratoberfläche variiert werden. In bevorzugten Ausführungsformen des Verfahrens bringt man das Enzym mittels physikalischer Adsorption, oder ionischer, koordinativer oder kovalenter Bindung auf die Oberfläche auf. Die kovalente Bindung an die Oberfläche kann über einen polymeren Abstandshalter vorzugsweise ausgewählt aus der Gruppe umfassend Polyethylenglycol, Polyvinylalkohol, Polyester und/oder Dextrane erfolgen. Es konnte gezeigt werden, dass Chymosin über Polyethylenglykol als polymeren Abstandhalter an eine Glasoberfläche gebunden wurde.

Abhängig von der Art und Stärke der Bindung des Enzyms an die Oberfläche kann dieses gegebenenfalls von der Oberfläche in ein umgebendes Lösungsmittel diffundieren. Der Bereich der Enzymdiffusion bestimmt die Ausdehnung des Reaktionsbereichs, in dem eine Spaltung des Biopolymers stattfinden kann. Für eine kontrollierte Abscheidung ist es vorteilhaft, dass die Spaltung des Biopolymers in der Nähe zur Oberfläche stattfindet, so dass sich die Spaltungsprodukte des Biopolymers mit geringerer Hydrophilie auf der Oberfläche des Substrats abscheiden und nicht, wie im Fall der Caseinmicellen möglich, in größerer Entfernung von der Oberfläche agglomerieren und in der Lösung ausfallen. Eine physikalische Adsorption, ionische, koordinative oder kovalente Bindung des Enzyms an eine Oberfläche kann dies zur Verfügung stellen.

Eine bevorzugte Ausführungsform ist die physikalische Adsorption des Enzyms an die Substratoberfläche. Eine physikalische Adsorption kann beispielsweise durch Auftropfen einer Lösung des Enzyms auf die Oberfläche und Antrocknen erzielt werden. Eine physikalische Adsorption kann eine Diffusion des Enzyms in das umgebende Lösungsmittel zur Verfügung stellen. Beispielsweise kann das wasserlösliche Chymosin in die Lösung diffundieren. Durch diese Beweglichkeit des Enzyms kann eine Reaktion mit dem Biopolymer auf der Oberfläche wie auch im Diffusionsbereich über der Oberfläche stattfinden. Diese Reaktionszone vergrößert sich mit steigender Reaktionszeit. Hierdurch wird die Reaktion des Enzyms mit dem Biopolymer solange erfolgen wie nicht gespaltenes Biopolymer verfügbar ist. Die Reaktion ist nicht selbstterminierend insofern als das Enzym auf der Oberfläche nicht insgesamt von abgeschiedenen Spaltprodukten bedeckt und damit inaktiviert wird. Dies erlaubt es, beispielsweise die Schichtdicke durch Variation der Reaktionsparameter wie Reaktionszeit und/oder Reaktionstemperatur zu beeinflussen. Eine hohe Enzymkonzentration und gute Bedingungen für die Enzymaktivität können zu einer beschleunigten und/oder erhöhten Schichtbildung führen. Generell führen mehrschichtige oder dickere Beschichtungen zu einer besseren Stabilität. Für Casein konnte gezeigt werden, dass die physikalische Adsorption von Chymosin an Glasoberflächen zu zusammenhängenden Caseinfilmen mit variabler Schichtdicke und Koaleszenzgrad führte.

Eine weitere bevorzugte Ausführungsform ist eine kovalente Bindung des Enzyms an die Oberfläche. Eine kovalente Immobilisierung ohne Spacer erlaubt nur eine geringe Enzymmobilität. Hierdurch kann eine Reaktion mit Biopolymer nur direkt an der Oberfläche stattfinden. Durch abgeschiedene Spaltungsprodukte wird das Enzym auf der Oberfläche bedeckt und steht nicht für weitere Spaltungsreaktionen zur Verfügung. Die Reaktion ist somit selbstterminierend und kann dünne Beschichtungen zur Verfügung stellen. So konnte für Casein mittels kovalenter Bindung von Chymosin an eine Oberfläche eine monomicellare Beschichtung hergestellt werden. Eine kovalente Bindung eines Enzyms kann über eine Funktionalisierung der Oberfläche bereitgestellt werden. Bei einer Funktionalisierung der Oberfläche werden spezielle, reaktive funktionelle Gruppen auf der Oberfläche gebunden, an die Enzyme spezifisch und selektiv binden können. Beispielsweise können Glasflächen mittels Silanen mit Amino- oder Epoxygruppen funktionalisiert werden.

In Abhängigkeit von der Art der Enzym-Aufbringung kann eine Beschichtung kontinuierlich oder auf definierten Bereichen der Oberfläche aufgebracht werden. In Ausführungsformen des Verfahrens bringt man das Enzym ganz- oder teilflächig auf die Oberfläche auf. Insbesondere mittels kovalenter Immobilisierung eines Enzyms auf einer Oberfläche kann eine selektive oder teilflächige Beschichtung zur Verfügung gestellt werden, da nur in Bereichen aufgebrachten Enzyms Spaltungsprodukte entstehen und abgeschieden werden. Dies erlaubt die Herstellung komplexer und strukturierter Beschichtungen. In vorteilhafter Weise konnte gezeigt werden, dass durch eine Abscheidung sehr kleiner Enzym-Aggregate eine Abscheidung von Casein-Einzelpartikeln erzielt werden kann.

Insgesamt kann das erfindungsgemäße Verfahren breite Möglichkeiten für eine kontrollierte Abscheidung von Proteinbeschichtungen zur Verfügung stellen. Insbesondere durch die Verwendung von Enzymen in direkter Nähe der Substratoberfläche finden die Spaltung und Abscheidung in unmittelbarer zum Substrat statt und einzelne Bereiche lassen sich gezielt und unter Kontrolle der Filmeigenschaften beschichten. Das Verfahren ermöglicht eine Steuerung des Abscheideprozesses, die bei üblichen Abscheideverfahren nicht erzielbar ist. Weiterhin ist die Verwendung biobasierter Materialien in vorteilhafter Weise rohstoffschonend, ungiftig und ermöglicht bei kovalenter Immobilisierung eine Wiederverwendbarkeit der Precursorlösungen.

Verfahrensparameter wie die Konzentration des Enzyms und des Biopolymers, die Reaktionszeit und die Temperatur, bei der die Abscheidungsreaktion durchgeführt wird, können abhängig von der gewünschten Schicht und/oder Schichtdicke variiert werden. In bevorzugten Ausführungsformen des Verfahrens kann das abzuscheidende Biopolymer in wässriger Lösung vorliegen, wobei die Konzentration des Biopolymers in der Lösung bevorzugt im Bereich von ≥ 0,01 g/L bis ≤ 50 g/L, vorzugsweise im Bereich von ≥ 0,1 g/L bis ≤ 10 g/L liegt. Weiter kann die Reaktionszeit zwischen Biopolymer und Enzym im Bereich von ≥ 1 min bis ≤ 240 min, vorzugsweise im Bereich von ≥ 5 min bis ≤ 60 min liegen. Weiter kann die Temperatur der Abscheidungsreaktion im Bereich von ≥ 0 °C bis ≤ 50 °C, vorzugsweise im Bereich von ≥ 30 °C bis ≤ 40 °C, liegen. Es konnte festgestellt werden, dass diese Verfahrensparameter zu guten Ergebnissen führten.

Durch geeignete Wahl der Aufbringung des Enzyms sowie der Verfahrensparameter sind Beschichtungen mit variabler Schichtdicke herstellbar. In Ausführungsformen des Verfahrens kann man eine Beschichtung mit einer Schichtdicke im Bereich von ≥ 10 nm bis ≤ 50 µm, vorzugsweise im Bereich von ≥ 20 nm bis ≤ 1 µm, aufbringen. Derartige Schichtdicken sind für Anwendungen als Beschichtung von medizinischen Implantaten, biologisch abbaubareren Materialien, essbaren Materialien, kolloidalen Partikeln oder als Klebeverbindung von zu verklebenden Flächen gut geeignet. Es konnte eine Abscheidung von sehr dünnen Monolagen an Casein erzielt werden. Die Schichtdicke kann im Bereich von ≥ 30 nm bis ≤ 300 nm liegen.

Enzyme können mittels Adsorption, ionischer, koordinativer oder kovalenter Bindung auf eine Vielzahl verschiedener Oberflächen aufgebracht werden. In vorteilhafter Weise sind mit dem erfindungsgemäßen Verfahren verschiedene Materialien beschichtbar. In Ausführungsformen kann die Oberfläche des Substrats aus Glas, Kunststoff, Metall, Holz oder Keramik ausgebildet sein. Das erfindungsgemäße Verfahren ist für die Beschichtung unterschiedlicher Substrate geeignet, beispielsweise zur Beschichtung von medizinischen Implantaten, oder kolloidalen Partikeln. Insbesondere Substrate mit komplexer Geometrie wie kleine medizinische Implantate sind durch das Verfahren mit Biopolymeren beschichtbar.

Ein weiterer Gegenstand der Erfindung betrifft einen beschichteten Gegenstand erhältlich durch das erfindungsgemäße Verfahren. Das erfindungsgemäße Verfahren kann ein Substrat bzw. einen Gegenstand bereitstellen, der mit einer biokompatiblen und/oder biologisch abbaubaren Beschichtung versehen ist. Insbesondere kann der Gegenstand ein medizinisches Implantat, ein biologisch abbaubareres Material, ein essbares Material, ein kolloidaler Partikel oder eine zu verklebende Fläche sein. Beispielsweise kann der beschichtete Gegenstand ein biologisch abbaubares Verpackungsmaterial sein, das mit einem Biopolymer wie Casein beschichtet ist, oder eine beschichtete essbare Verpackung oder Lebensmittel. Eine zu verklebende Fläche kann eine Fläche eines Objektes sein, wobei auf diese ein Biopolymer wie Casein abgeschieden wird, so dass zwischen zwei Objekten eine verbindende Caseinschicht entsteht, die als in-situ Bio-Klebstoff fungieren kann. Es konnte gezeigt werden, dass durch eine über Reaktion mit Chymosin präzipitierte Caseinschicht Glasoberflächen und Metalloberflächen haftend miteinander verklebt werden konnten.
Durch das erfindungsgemäße Verfahren kann beispielsweise eine biokompatible Beschichtung auf einem Implantat bereitgestellt werden, mit dem ein Implantat herstellbar ist, das durch seine Biokompatibilität eine verbesserte Verträglichkeit aufweist. Unter einem Implantat ist erfindungsgemäß ein Substrat zu verstehen, dass dafür geeignet ist, in einen Patienten implantiert zu werden. Beispiele für Implantate sind Katheter, Osteosynthesematerial, Endoprothesen, Nägel, Schrauben und/oder Drähte, Herzklappen, künstliche Blutgefäße und Shunts, gesichtschirurgische oder plastische Implantate, Mittelohrimplantate oder Dentalimplantate.
Eine biokompatible Beschichtung ist insbesondere in Verbindung mit einer Beschichtung von Objekten komplexer Geometrie im Bereich der medizinischen Implantologie von Vorteil. Biologisch abbaubare Beschichtung ermöglichen selbstabbaubare Implantate. Durch die Abbaubarkeit kann beispielsweise bei temporären Implantaten auf eine zweite Operation zur Entfernung des Implantats verzichtet werden. Auch im Bereich der Mikrotechnologie sind durch das erfindungsgemäße Verfahren beschichtete Gegenstände für eine Verwendung im biologischen Umfeld vorteilhaft verwendbar.

Erfindungsgemäß umfasst der Gegenstand eine Casein-Beschichtung ausgebildet aus Casein-Micellen, wobei ein hydrophiler Teil des κ-Casein an der Stelle der Phenylalanin¹⁰⁵-Methionin¹⁰⁶-Bindung abgespalten wurde. Die Beschichtung ist durch das erfindungsgemäße Verfahren unter Verwendung von Chymosin und/oder Pepsin herstellbar. Eine Casein-Beschichtung ausgebildet aus Casein-Micellen, umfassend im Wesentlichen hydrophobes α_{S1}-Casein, α_{S2}-Casein, β-Casein und para-κ-Casein, wobei das hydrophile Caseinmakropeptid abgespalten wurde, kann gegenüber Beschichtungen mit nicht gespaltenem Casein eine höhere Hydrophobizität und damit höhere Beständigkeit der Caseinbeschichtung gegenüber Wasser zur Verfügung stellen. Die Casein-Beschichtung kann vollflächig oder teilflächig insbesondere auf definierten Bereichen der Oberfläche des Gegenstandes aufgebracht sein. In Ausführungsformen kann die Casein-Beschichtung eine Schichtdicke im Bereich von ≥ 10 nm bis ≤ 50 µm, vorzugsweise im Bereich von ≥ 20 nm bis ≤ 1 µm, aufweisen. Insbesondere kann die Casein-Beschichtung eine monomicellare Beschichtung sein.

Ebenfalls offenbart ist eine Beschichtungszusammensetzung, enthaltend ein Biopolymer und wenigstens eine Komponente ausgewählt aus der Gruppe umfassend Bindemittel, Füllstoffe, Pigmente und/oder Additive sowie optional ein Lösemittel.
Unter dem Begriff "Beschichtungszusammensetzung" ist im Sinne der vorliegenden Erfindung eine flüssig-, pasten- oder pulverförmige Zusammensetzung zu verstehen, die, auf ein Substrat oder Untergrund aufgetragen, eine Beschichtung ergibt. Die Oberfläche des Substrats kann aus Glas, Kunststoff, Metall, Holz oder Keramik ausgebildet sein. Bevorzugt ist eine flüssigförmige Beschichtungszusammensetzung enthaltend ein Lösemittel. Diese wird als Beschichtungslösung bezeichnet. Die Beschichtungslösung ist vorzugsweise eine wässrige Lösung. Das Lösemittel ist entsprechend vorzugsweise Waser.
Das Biopolymer ist vorzugsweise ein Protein, Proteinkomplex oder Proteingemisch. Das Protein kann insbesondere Casein oder Fibrinogen sein. Die Konzentration des Biopolymers in der Beschichtungszusammensetzung insbesondere der Beschichtungslösung kann im Bereich von ≥ 0,01 g/L bis ≤ 50 g/L, vorzugsweise im Bereich von ≥ 0,1 g/L bis ≤ 10 g/L, liegen.

Die weiteren Komponenten der Beschichtungszusammensetzung oder Beschichtungslösung können grundsätzlich dem Fachmann bekannte Bestandteile sein, beispielsweise beschrieben in B. Müller, U. Poth, "Lackformulierungen und Lackrezeptur", 3. Auflage 2009, Vincentz Network. Beispielsweise kann die Beschichtungszusammensetzung oder Beschichtungslösung Pigmente enthalten. Bevorzugte Pigmente sind anorganische Partikel, insbesondere Metalloxide wie Titandioxid. Diese können als fein verteilte bzw. dispergierte Partikel mit einer Größe im Bereich von 0,01 µm bis 1 µm in der Lösung bzw. in der Beschichtung vorliegen. TiO₂ ist zur Verbesserung der Weiße oder Opazität einer Beschichtung verwendbar.

Die Beschichtungszusammensetzung ist herstellbar, indem das Biopolymer, insbesondere Casein, und die wenigstens eine Komponente ausgewählt aus der Gruppe umfassend Bindemittel, Füllstoffe, Pigmente und/oder Additive vermischt oder in einem Lösemittel gelöst oder dispergiert werden. Beispielsweise kann Titandioxid in einer Caseinlösung dispergiert werden.

Die Beschichtungszusammensetzung ist zur Beschichtung von Oberflächen verwendbar. Insbesondere eine Beschichtungslösung ist in Verfahren zur Beschichtung von Oberflächen durch enzymatische Reaktion eines Biopolymers verwendbar. So kann man a) ein Enzym auf die Oberfläche eines Substrats aufbringen, und b) das Enzym mit der Beschichtungslösung in Kontakt bringen, wobei das Enzym das Biopolymer spaltet, wobei durch die Spaltung wenigstens zwei Spaltungsprodukte des Biopolymers mit unterschiedlicher Löslichkeit in dem Lösungsmittel entstehen, und wenigstens ein Spaltungsprodukt des Biopolymers mit geringerer Löslichkeit auf der Oberfläche des Substrats abgeschieden wird. Enthält die Beschichtungslösung beispielsweise Titandioxid-Partikel, können diese von abgeschiedenem Protein umhüllt sein und einer ansonsten im Wesentlichen farblosen Proteinschicht eine weiße oder sich im Farbton Weiß nähernde Färbung verleihen.

Beispiele und Figuren, die der Veranschaulichung der vorliegenden Erfindung dienen, sind nachstehend angegeben.

Hierbei zeigen die Figuren:
- Figur 1: zeigt REM-Aufnahmen von Casein-Schichten aufgebracht mittels adsorbiertem Chymosin auf einem Glasträger. Figur 1a zeigt eine Schicht aufgebracht aus einer wässrigen Casein-Lösung mit 1 g/L Casein, Figur 1b zeigt eine Schicht aufgebracht aus einer wässrigen Casein-Lösung mit 10 g/L Casein. Die Schichtgrenzen zur Glasoberfläche sind jeweils durch Pfeile gekennzeichnet.
- Figur 2: zeigt EDX-REM-Bestimmungen von kovalent auf einem Glasträger aufgebrachtem Chymosin. Figur 2a zeigt die REM-Aufnahme der Oberfläche, wobei mit Punkt 1 ein Bereich der unbeschichteten Oberfläche gekennzeichnet ist und ein Bereich mit vermuteter Enzymabscheidung mit Punkt 2 gekennzeichnet ist. Die Figur 2b zeigt die EDX-Spektren der Punkte 1 und 2.
- Figur 3: zeigt REM-Aufnahmen der Oberfläche eines Glasträgers mit immobilisiertem Chymosin in Figur 3a, und nach der Abscheidung von Casein auf Bereiche immobilisierten Chymosins in Figur 3b.
- Figur 4: zeigt eine REM-Aufnahme der Oberfläche eines Glasträgers mit einer Beschichtung aufgebracht aus einer wässrigen Casein-Lösung mit 10 g/L Casein enthaltend dispergierte Titandioxid-Partikel.
- Figur 5: zeigt eine REM-Aufnahme eines Glasobjektträgers, der mit Chymosin-Lösung beschichtet wurde.
- Figur 6: zeigt eine REM-Aufnahme eines Glasobjektträgers nach dem Verkleben mit einer wässrigen Casein-Lösung. Die Figuren 6a, b und c zeigen jeweils verschieden Vergrößerungen.

### Materialien

Casein aus Kuhmilch und Chymosin wurden von Sigma-Aldrich bezogen und ohne weitere Reinigung verwendet. Die weiteren Chemikalien wurden von Fluka, Sigma-Aldrich oder ABCR bezogen und ebenfalls ohne Reinigung verwendet.

### Beispiel 1

### Ausbildung einer Casein-Schicht mittels adsorbiertem Enzym auf einem Glasträger

Glasobjektträger (Carl Roth) wurden mit einer Mischung von 70 % zu 30 % (v/v) von Schwefelsäure zu Wasserstoffperoxid gereinigt und mit deionisiertem Wasser gewaschen. Danach wurde eine Lösung von 1,7 mg/ml Chymosin (bezogen auf das reine Enzym) in deionisiertem Wasser auf die Oberfläche getropft und bei 20 °C getrocknet. Die Glasobjektträger mit adsorbiertem Chymosin wurden jeweils in eine wässrige Casein-Lösung einer Konzentration von 1 g/L, 5 g/L oder 10 g/L Casein bei pH 3 und 40 °C gestellt. Nach 20, 40 oder 60 Minuten wurden die Objektträger aus der Casein-Lösung entnommen, mit deionisiertem Wasser gewaschen und getrocknet.

Die erhaltenen Casein-Filme wurden mittels Elektronenmikroskopie unter Verwendung eines Zeiss "Neon 40" Rasterelektronenmikroskops ausgestattet mit EDX-Detektor untersucht. REM-Aufnahmen der Proben wurden durch Verwendung des InLens-Detektors (Sekundärelektronen) und des SE-Detektors (Sekundär-und Rückstreuelektronen) bei einer Beschleunigungsspannung von 2 kV erhalten.

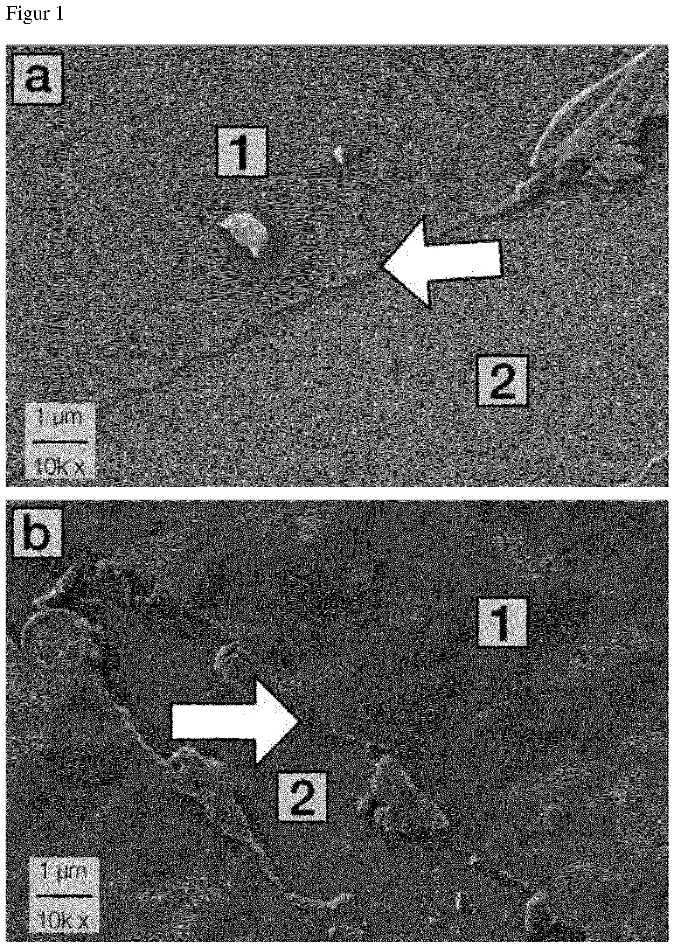

Die Figur 1 zeigt die REM-Aufnahmen der mittels adsorbiertem Chymosin auf Glasträger aufgebrachten Casein-Schichten. Die Figur 1a zeigt eine Schicht 1 aufgebracht aus einer wässrigen Casein-Lösung mit 1 g/L Casein und einer Reaktionszeit von 60 Minuten. Diagonal über die Aufnahme ist eine durch Abkratzen des Caseins in unteren rechten Bereich der Aufnahme entstandene Schichtgrenze zu erkennen. Die Glasfläche ist mit 2 gekennzeichnet. Figur 1b zeigt eine Schicht 1 aufgebracht aus einer wässrigen Casein-Lösung mit 10 g/L Casein und einer Reaktionszeit von 60 Minuten. Diagonal über die Aufnahme ist ein freigekratzter Streifen der Glasoberfläche 2 zu erkennen. Wie einem Vergleich der Figuren 1a und 1b entnommen werden kann, war die aufgebrachte Schicht aus der 1 g/L Casein-Lösung wesentlich dünner als die Schicht aufgebracht aus der 10 g/L Casein-Lösung. Beide REM-Aufnahmen zeigten jeweils homogene Filme mit voller Koaleszenz der Casein-Micellen. Anhand der REM-Aufnahmen wurde eine Dicke der erhaltenen Casein-Schichten im Bereich von 10 nm bis 30 nm bzw. 100 nm bis 250 nm geschätzt.

Dies zeigt, dass bei den verwendeten Konzentrationen und Reaktionszeiten jeweils vollflächige, homogene Casein-Schichten variabler Dicke abgeschieden wurden. Die steigende Dicke der Casein-Schicht mit steigender Casein-Menge und Zeit zeigt weiter, dass die Reaktion nicht selbstterminierend ist, sondern abläuft, solange adsorbiertes Chymosin und Casein-Micellen in der Nähe der Oberfläche verfügbar sind.

### Beispiel 2

### Bestimmung der Beständigkeit der Casein-Beschichtung gegenüber Wasser

Zur Überprüfung der enzymatisch-katalysierten Spaltungsreaktion in einen hydrophilen und einen hydrophoben Teil des Caseins zur Ausbildung der Casein-Beschichtung, wurden Casein-Beschichtungen mit und ohne Verwendung des Enzyms auf Glasobjektträger aufgebracht.

Vier Glasobjektträger (Carl Roth) wurden mit einer Mischung von 70 % zu 30 % (v/v) von Schwefelsäure zu Wasserstoffperoxid gereinigt und mit deionisiertem Wasser gewaschen. Anschließend wurde auf zwei der vier Glasobjektträger eine Lösung von 1,7 mg/ml Chymosin in deionisiertem Wasser auf die Oberfläche getropft und bei 20°C getrocknet. Alle vier Glasobjektträger wurden anschließend in eine wässrige Casein-Lösung einer Konzentration von 10 g/L Casein bei pH 3 und 40 °C gestellt. Nach 60 Minuten wurden die Objektträger aus der Casein-Lösung genommen und bei 20 °C getrocknet. Anschließend wurden 2 der 4 Glasobjektträger, wobei einer Enzym aufwies und einer ohne Enzym war, mit deionisiertem Wasser gewaschen und erneut bei 20 °C getrocknet. Die anderen beiden Glasobjektträger wurden nicht gewaschen.

Alle 4 Glasobjektträger wurden mittels Elektronenmikroskopie unter Verwendung eines Zeiss "Neon 40" Rasterelektronenmikroskops ausgestattet mit EDX-Detektor untersucht. REM-Aufnahmen wurden durch Verwendung des InLens-Detektors (Sekundärelektronen) und des SE-Detektors (Sekundär-und Rückstreuelektronen) bei einer Beschleunigungsspannung von 2 kV erhalten. Beide nicht-gewaschenen Proben zeigten eine kontinuierliche Beschichtung und wiesen NaCl-Kristalle auf der Oberfläche auf, die aus der verwendeten Enzym-Lösung und der pH-Wert-Einstellung mit HCl und NaOH stammten. Der gewaschene Glasobjektträger ohne Enzym wies hingegen keine Beschichtung mehr auf. Die konventionell aufgetrocknete Caseinschicht wurde wie erwartet abgespült. Der gewaschene Glasobjektträger mit Enzym zeigte wiederum eine durchgängige Beschichtung mit einem Casein-Film, währen die Salzkristalle und eventuell vorhandenes nicht-gespaltenes Caseins abgewaschen wurde.

Dies zeigt, dass die unter enzymatischer Spaltung abgeschiedene Caseinschicht im Gegensatz zu der konventionell aufgetrockneten Caseinschicht eine höhere Wasserfestigkeit aufwies und stabil auf der Oberfläche verblieb. Dies wird auf eine höhere Hydrophobizität des enzymatisch abgeschiedenen Teils des Caseinproteins zurückgeführt.

### Beispiel 3

### Bestimmung der Hydrophobizität der Casein-Beschichtung

Glasobjektträger (Carl Roth) wurden mit einer Mischung von 70 % zu 30 % (v/v) von Schwefelsäure zu Wasserstoffperoxid gereinigt und mit deionisiertem Wasser gewaschen. Anschließend wurde auf die Oberfläche von einem gereinigten Glasobjektträger eine Lösung von 1,7 mg/ml Chymosin in deionisiertem Wasser aufgetropft und bei 20 °C getrocknet. Der Glasobjektträger mit dem adsorbiertem Chymosin wurde anschließend in eine wässrige Casein-Lösung einer Konzentration von 10 g/L Casein bei pH 3 und 40 °C gestellt. Nach 60 Minuten wurde der Objektträger aus der Casein-Lösung genommen, mit deionisiertem Wasser gewaschen und getrocknet. Auf die Oberfläche von einem weiteren gereinigten Glasobjektträger wurde eine wässrige Casein-Lösung einer Konzentration von 10 g/L Casein, pH 3, getropft und bei 40 °C getrocknet.

Anschließend wurde die Hydrophobizität der enzymatisch sowie der konventionell aufgebrachten Casein-Beschichtungen im Vergleich zur Hydrophobizität der reinen Glasfläche über eine Messung des Kontaktwinkels von Wasser auf der jeweiligen Beschichtung bestimmt. Die Kontaktwinkelmessungen von festsitzenden Tropfen deionisierten Wassers wurden mit einem "Kontaktwinkel-Messsystem G10" (KRÜSS) bestimmt. Der Kontaktwinkel wurde eine Sekunde nach der Platzierung der Tropfen auf dem jeweiligen Träger gemessen. Die folgende Tabelle 1 zeigt die gemessenen Kontaktwinkel. Die angegebenen Kontaktwinkel sind jeweils Durchschnittswerte aus drei Einzelmessungen.

**Tabelle 1: Kontaktwinkel**

| Probe | Kontaktwinkel [°] |
|---|---|
| gereinigte Glasfläche | 15,5 ± 1,5 |
| konventionell aufgetropfte Caseinschicht | 54,7 ± 8,7 |
| enzymatisch-katalysiert aufgebrachte Caseinschicht | 75,3 ± 1,1 |

Die Tabelle 1 zeigt, dass der Kontaktwinkel der enzymatisch aufgebrachten Beschichtung von gespaltenem Casein ca. 20 ° über dem Wert des Kontaktwinkels der aufgetropften Beschichtung mit nicht gespaltenem Caseins liegt. Die hohe Standardabweichung im Fall des nicht-gespaltenen Caseins ist auf eine teilweise Auflösung des Films während der Messung zurückzuführen. Dies bestätigt die höhere Hydrophobizität der enzymatisch aufgebrachten Caseinbeschichtung.

### Beispiel 4

### Ausbildung einer Casein-Schicht mittels kovalenter Immobilisierung von Chymosin auf Glasflächen

Glasobjektträger (Carl Roth) wurden mit einer Mischung von 70 % zu 30 % (v/v) von Schwefelsäure zu Wasserstoffperoxid gereinigt und mit deionisiertem Wasser gewaschen. Für eine kovalente Immobilisierung von Chymosin auf der Glasfläche wurde diese zunächst mit Epoxygruppen funktionalisiert. Hierzu wurde eine 10 %ige (w/w) Lösung von 3-Glycidoxypropyltrimethoxysilan (GOPS) in einer 80/20 (w/w) Mischung von Ethanol (EtOH) und Wasser unter alkalischen Bedingungen unter Verwendung von 1 % (w/w) Triethylamin (TEA) hergestellt. Die Glasobjektträger wurden in die Lösung getaucht und die Reaktionsmischung wurde vorsichtig für 1 Stunde bei Raumtemperatur (20±2 °C) gerührt und danach für weitere 10 Minuten ungerührt stehen gelassen. Anschließend wurden die Deckgläser mit Ethanol gespült, um unspezifisch adsorbiertes 3-Glycidoxypropyltrimethoxysilan zu entfernen. Die Gläser wurden dann in einem Ofen bei 110 °C für 1 Stunde gehärtet, mit reichlichen Mengen an Ethanol und deionisiertem Wasser gespült, in einem Abzug trocknen gelassen und in einem Exsikkator gelagert.

Für die Immobilisierung wurden 6 mg Chymosin Formulierung (Sigma-Aldrich) in 100 ml Reaktionspuffer, einer Mischung von 1 Teil Phosphatpuffer (Sigma Aldrich) und 19 Teilen entionisiertem Wasser, pH 7,4, gelöst. Die Immobilisierungsreaktion wurde unter leichtem Rühren bei Raumtemperatur durchgeführt. Der Fortschritt der Immobilisierung wurde durch die Entnahme von Proben (500 µl) des Überstands der Lösung verfolgt, die anschließend mit 500 µl einer 1%igen (w/v) Hämoglobin-Lösung in Reaktionspuffer (Dinatriumhydrogenphosphat / Zitronensäure, pH = 3) gemischt wurden, um die Enzymaktivität zu bestimmen. Die Reaktion des Chymosin mit Hämoglobin wurde bei 40 °C für 30 min in einem Wasserbad bei pH 3,5 durchgeführt, und anschließend durch Zugabe von 500 µl 10%iger (w/v) Trichloressigsäure-Lösung gestoppt. Anschließend wurden die Proben bei 13.000 rpm für 15 min zentrifugiert und nach der Zentrifugation die Extinktion des Überstandes bei 325 nm im Vergleich zu Hämoglobin-Kontrollproben gemessen. Nach 4 Tagen wurde der Immobilisierungs-Vorgang gestoppt und die Glasobjektträger wurden mit deionisiertem Wasser gespült und in einem Exsikkator gelagert.

Die Glasobjektträger mit kovalent gebundenem Chymosin wurden in eine wässrige Casein-Lösung einer Konzentration von 10 g/L Casein bei pH 3 und 40 °C gestellt. Nach 60 Minuten wurde der Objektträger entnommen, mit deionisiertem Wasser gewaschen und getrocknet.

Glasobjektträger mit kovalent an die epoxy-funktionalisierte Oberfläche gebundenem Chymosin und Glasobjektträger mit darauf abgeschiedenem Casein wurden mittels Elektronenmikroskopie unter Verwendung eines Zeiss "Neon 40" Rasterelektronenmikroskops ausgestattet mit EDX-Detektor untersucht. Bilder der Proben wurden durch Verwendung des InLens-Detektors (Sekundärelektronen) und des SE-Detektors (Sekundär-und Rückstreuelektronen) bei einer Beschleunigungsspannung von 2 kV erhalten.

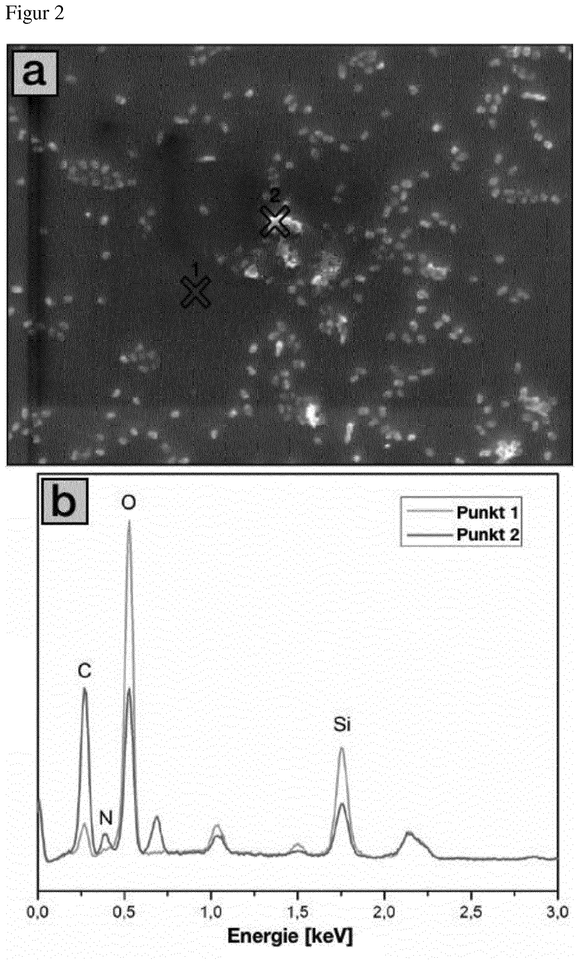

Die REM-Aufnahmen der Oberfläche nach der Enzym-Immobilisierung zeigten Bereiche mit partikulären Strukturen einer Größe von etwa 500 nm, von denen angenommen wurde, dass es sich um agglomeriertes Enzym handelte. Um diese Annahme zu bestätigen, wurden EDX-Messungen der reinen Oberfläche und der Bereiche der vermuteten Enzym-Abscheidung durchgeführt. Die Figur 2 zeigt die EDX-REM-Bestimmungen. Hierbei zeigt die Figur 2a die REM-Aufnahme der Oberfläche. Mit Punkt 1 ist ein Bereich der unbeschichteten Oberfläche gekennzeichnet und ein Bereich mit vermuteter Enzymabscheidung ist mit Punkt 2 gekennzeichnet. Die Figur 2b zeigt die EDX-Spektren der Punkte 1 und 2. Das Spektrum des Messpunkts 1 zeigte ein Sauerstoff/Silizium-Verhältnis von etwa 2:1 und Spuren von Kohlenstoff. Diese wurden der Siloxan-Funktionalisierung zugeschrieben. Die vermuteten Enzym-Bereiche zeigten einen hohen Kohlenstoffanteil und zusätzlich war Stickstoff nachweisbar. Das Verhältnis der Elemente stand in Einklang mit Proteinstrukturen und bestätigte das Vorliegen von Chymosin auf der Oberfläche.

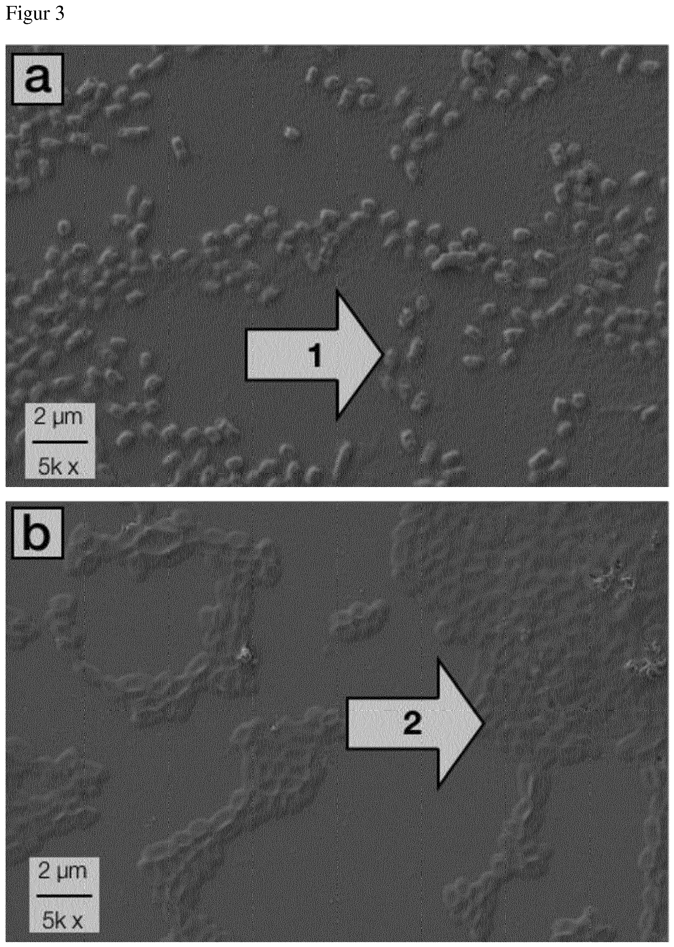

Die in Figur 3 gezeigten REM-Aufnahmen der Oberfläche des Glasträgers zeigten partikuläre Strukturen kovalent immobilisierten Chymosins in Figur 3a, wobei eine partikuläre Struktur ist mit einem Pfeil 1 gekennzeichnet ist, und in Figur 3b eine spezifische Beschichtung der Bereiche immobilisierten Enzyms durch Abscheidung von Casein auf dem Enzym, wobei die übrigen Bereiche der Oberfläche unbeschichtet blieben. Ein Bereich auf dem Enzym abschiedenen Caseins ist mit einem Pfeil 2 gekennzeichnet. Es wurde ein sehr dünner Caseinfilm abgeschieden, unter dem die partikulären Strukturen des immobilisierten Chymosins weiterhin gut erkennbar blieben. Es wird daher angenommen, dass sich auf dem kovalent auf der Oberfläche gebundenen Enzym lediglich eine monomicellare Schicht von Casein ausgebildet hatte. Es wird vermutet, dass das abgeschiedene gespaltene Casein das Enzym bedeckt und damit weitere Reaktionen in diesem spezifischen Bereich verhindert.

Dieses Ergebnis zeigt, dass die Aktivität des kovalent an die Oberfläche gebundenen Enzyms ausreicht, die Spaltungsreaktion zu katalysieren und stellt die Möglichkeit spezifischer teilflächiger Proteinbeschichtungen zur Verfügung.

### Beispiel 5

### Abscheidung einer Casein und Titandioxid enthaltenden Beschichtungslösung mittels adsorbiertem Enzym auf einem Glasträger

In 100 mL einer wässrigen Casein-Lösung mit einer Konzentration von 10 g/L Casein, pH 3, wurden 2 g TiO₂ (Sigma-Aldrich) dispergiert, wobei die Partikel eine geschätzte Größe im Bereich von 200 nm bis 400 nm aufwiesen. Das Dispergieren erfolgte über 30 Minuten bei 700 U/min in einem Dissolver.

Glasobjektträger wurden mit einer Mischung von 70 % zu 30 % (v/v) von Schwefelsäure zu Wasserstoffperoxid gereinigt und mit deionisiertem Wasser gewaschen. Danach wurde eine Lösung von 1,7 mg/ml Chymosin, bezogen auf das reine Enzym, in deionisiertem Wasser auf die Oberfläche getropft und bei 20 °C getrocknet.

Anschließend wurde die Dispersion auf dem Glasobjektträger mit adsorbiertem Chymosin abgeschieden, indem der Glasobjektträger bei 40 °C für 60 Minuten in die Caseinlösung mit dispergiertem TiO₂ gestellt wurde. Danach wurde der Objektträger aus der Casein-Lösung entnommen, mit deionisiertem Wasser gewaschen und getrocknet.

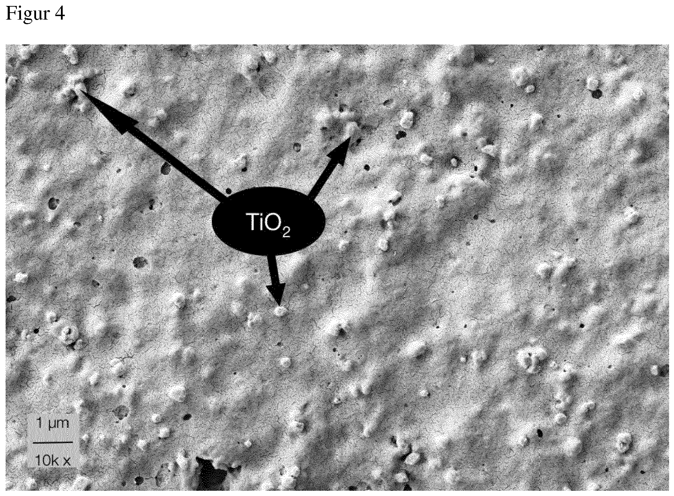

Der erhaltene Casein-Film wurde mittels Elektronenmikroskopie untersucht. Die Figur 4 zeigt die REM-Aufnahmen der aufgebrachten Casein-Schicht. Die in Figur 4 gezeigte REM-Aufnahme zeigte eine durchgängige Beschichtung mit einer weißlichen Färbung. Weiterhin wies die Beschichtung partikuläre Strukturen auf, die mit Casein beschichteten Titandioxid-Partikeln zugeschrieben wurden. Es wurde angenommen, dass die dispergierten Titandioxid-Partikel von Casein umhüllt und abgeschieden wurden.

Dieses Ergebnis zeigt, dass mit dem erfindungsgemäßen Verfahren auch Beschichtungslösungen enthaltend Casein und übliche Lack-Komponenten wie Titandioxid abgeschieden werden können.

Insgesamt zeigen die Beispiele, dass das Enzym mittels physikalischer Adsorption oder kovalenter Bindung auf die Oberfläche aufbringbar ist, wobei mittels physikalischer Adsorption dickere Beschichtungen kontrollierter Schichtdicken abscheidbar sind, während kovalent an die Oberfläche gebundenes Enzym die Möglichkeit teilflächiger Proteinbeschichtungen bietet.

### Beispiel 6

### Abscheidung kleinflächiger Enzymaggregate mittels kovalenter Immobilisierung von Chymosin

Glasobjektträger (VWR) wurden wie in Beispiel 4 beschrieben mit einer Mischung von Schwefelsäure und Wasserstoffperoxid gereinigt und mit einer 10 %igen Lösung von 3-Glycidoxypropyltrimethoxysilan (GOPS) epoxy-funktionalisiert. Anschließend wurde unspezifisch adsorbiertes GOPS entfernt und die beschichtete Glasoberfläche bei 110 °C für 1 Stunde gehärtet.

Chymosin wurde in Form von Labpulver über den europäischen Lieferanten BICHSEL-AG (Schweiz) von RENCO NEW ZEALAND bezogen. Der Anteil an Chymosin betrug nach Herstellerangabe 5 % (w/w). Um den Chymosin-Anteil und damit die Effizienz der enzymatischen Reaktion zu erhöhen, wurde der Salzgehalt durch Ultrafiltration verringert. Hierfür wurde das Labpulver in deionisiertem Wasser gelöst und drei Mal für zwei Stunden bei 5.000 x g und 25 °C über Amicon® Ultra-15 Zentrifugenfiltermittel bis zu einem Chymosinanteil von 67 % (w/w) zentrifugiert.

Chymosin-Lösungen einer Enzymmenge von 60 µg/mL wurden in 25 mM und 1 M Phosphatpufferlösungen bei pH 7,4 hergestellt und bei Raumtemperatur während 93 h auf den beschichteten Glasobjektträgern immobilisiert, wobei die Aktivität gemessen wurde wie in Beispiel 4 beschrieben.

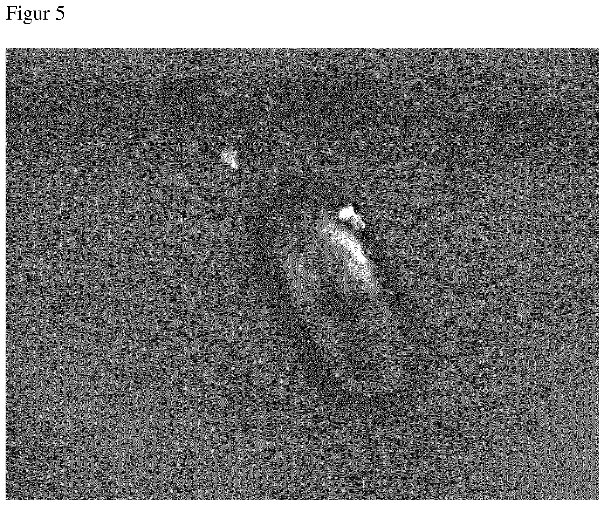

Die Glasobjektträger mit kovalent gebundenem Chymosin wurden in eine wässrige Casein-Lösung einer Konzentration von 10 g/L Casein bei pH 3 und 40 °C gestellt. Nach 60 Minuten wurde der Objektträger entnommen, mit deionisiertem Wasser gewaschen und getrocknet. Die erhaltenen Caseinbeschichtungen wurden mittels Elektronenmikroskopie (REM) unter Verwendung eines Zeiss "Neon 40" Rasterelektronenmikroskops untersucht. Aufnahmen der Proben wurden unter Verwendung des SE2-Detektors mit hohem topografischem Kontrast bei einer Beschleunigungsspannung von 2 kV aufgenommen. Figur 5 zeigt eine REM-Aufnahme eines Glasobjektträgers, der mit Chymosin in einer 25 mM Phosphatpufferlösung beschichtet wurde. Es wurde festgestellt, dass sich auf diesen Glasträgern ca. 500 nm große Aggregate aus Enzym bildeten, welche eine Abscheidung von Casein-Einzelpartikeln initiieren, wenn die Aggregate isoliert vorlagen. Wie die Figur 5 weiter zeigt, wurde eine Abscheidung von einzelnen Casein-Mizellen in einem Umkreis von < 1 µm um die Enzymaggregate herum beobachtet. Bei den Glasträgern, die mit Chymosin in einer 1 M Phosphatpufferlösung beschichtet wurden, wurden sehr viel kleinere Enzymagglomerate angebunden, die eine Größe von ca. 15 nm bis 25 nm aufwiesen. Dies zeigt, dass sich je nach aufgrund der variablen Pufferkonzentration vorliegenden Ionenstärke größere oder kleinere Aggregate bildeten. Bei höheren Ionenstärken konnten hierbei kleinere Aggregate erzielt werden.

### Beispiel 7

### Kovalente Bindung des Enzyms an die Oberfläche über Glutaraldehyd

Mit Schwefelsäure und Wasserstoffperoxid gereinigte Glasobjektträger (VWR) wurden amino-funktionalisiert. Hierzu wurde eine 10 %ige (w/w) Lösung von Aminopropyltriethoxysilan (APS) in einer 80:20 (w/w) Mischung von Ethanol (EtOH) und Wasser unter neutralen Bedingungen hergestellt. Die Glasobjektträger wurden für 2 Stunden bei Raumtemperatur (20±2 °C) in die Lösung getaucht. Anschließend wurde mit Ethanol gespült, um unspezifisch adsorbiertes APS zu entfernen. Die Gläser wurden dann in einem Ofen bei 110 °C für 1 Stunde gehärtet, mit reichlichen Mengen an Ethanol und deionisiertem Wasser gespült, in einem Abzug trocknen gelassen und bis zur weiteren Verwendung in einem Exsikkator gelagert. Diese APS-aktivierten Glasträger wurden in einem zweiten Schritt für 17 Stunden bei Raumtemperatur (20±2 °C) mit einer 15 %igen (w/w) Lösung von Glutaraldehyd in einer Mischung von 1 Teil Phosphatpuffer (Sigma Aldrich) und 19 Teilen entionisiertem Wasser, pH 7,4, inkubiert, wodurch eine endständige Aldehydgruppe vorlag.

Eine Chymosin-Lösungen einer Konzentration von 60 µg/mL in einer Mischung von 1 Teil Phosphatpuffer und 19 Teilen entionisiertem Wasser, pH 7,4, wurde hergestellt und bei Raumtemperatur während 24 h auf den beschichteten Glasobjektträgern immobilisiert, wobei die Aktivität wie in Beispiel 4 beschrieben überwacht wurde.

Die Glasobjektträger mit kovalent gebundenem Chymosin wurden in eine wässrige Casein-Lösung einer Konzentration von 10 g/L Casein bei pH 3 und 40 °C gestellt. Nach 60 Minuten wurde der Objektträger entnommen, mit deionisiertem Wasser gewaschen und getrocknet. Die erhaltenen Caseinbeschichtungen wurden mittels Elektronenmikroskopie (REM) untersucht.

Es wurde festgestellt, dass sich auf der amino-funktionaliserten Oberfläche der Glasträger eine eher kontinuierliche Enzymverteilung ausbildete. Die Ausbildung kleiner Aggregate von ca. 50 nm wird auf die verwendete Immobilisierungsmethode zurückgeführt. Auf diese nicht isoliert vorliegenden die Enzym-Aggregate wurde eine filmartige, sehr dünne Monolage an Casein abgeschieden.

### Beispiel 8

### Kovalente Bindung des Enzyms an die Oberfläche über Polyethylenglykol als polymeren Abstandshalter

Gereinigte Glasobjektträger wurden wie in Beispiel 7 beschrieben mit Aminopropyltriethoxysilan (APS) amino-funktionalisiert. Diese APS-aktivierten Glasträger wurden in einem zweiten Schritt für 24 Stunden bei Raumtemperatur (20±2 °C) mit einer 0,25g/L Lösung von Polyethylenglykol-Diglycidylether (Mₙ = 20.000 g/mol, Creative PEGWorks, USA) in 100 mM Phosphatpuffer inkubiert. Hierdurch wurde eine endständige Epoxygruppe über Polyethylenglykol als polymeren Abstandhalter an die Glasoberfläche gebunden.

Eine Chymosin-Lösungen einer Konzentration von 60 µg/mL in einer Mischung von 1 Teil Phosphatpuffer und 19 Teilen entionisiertem Wasser, pH 7,4, wurde hergestellt und bei Raumtemperatur während 24 h auf den beschichteten Glasobjektträgern immobilisiert, wobei die Aktivität wie in Beispiel 4 beschrieben überwacht wurde.

Die Glasobjektträger mit kovalent gebundenem Chymosin wurden in eine wässrige Casein-Lösung einer Konzentration von 10 g/L Casein bei pH 3 und 40 °C gestellt. Nach 60 Minuten wurde der Objektträger entnommen, mit deionisiertem Wasser gewaschen und getrocknet. Die erhaltene Caseinbeschichtung wurde mittels Elektronenmikroskopie (REM) untersucht.

Es wurde festgestellt, dass das Enzym über Polyethylenglykol als polymeren Abstandhalter erfolgreich an die Glasoberfläche gebunden wurde. Weiter wurde beobachtet, dass bei der Verwendung des Spacers unter den beschriebenen Bedingungen Hybridstrukturen entstanden, wobei ein innerer "Ring" mit einer kontinuierlichen, filmartigen Casein-Abscheidung und zusätzlich eine Abscheidung einzelner Mizellen, also von Einzelpartikeln, in einem äußeren "Ring" beobachtet wurde.

### Beispiel 9

### Verkleben von Glasobjektträgern über gespaltenes Casein

Chymosin wurde in Form von Labpulver über den europäischen Lieferanten BICHSEL-AG (Schweiz) von RENCO NEW ZEALAND bezogen. Der Anteil an Chymosin betrug nach Herstellerangabe 5 % (w/w). Um den Chymosin-Anteil und damit die Effizienz der enzymatischen Reaktion zu erhöhen, wurde der Salzgehalt durch Ultrafiltration verringert. Hierfür wurde das Labpulver in deionisiertem Wasser gelöst und drei Mal für zwei Stunden bei 5.000 x g und 25 °C über Amicon® Ultra-15 Zentrifugenfiltermittel bis zu einem Chymosinanteil von 67 % (w/w) zentrifugiert.

Glasobjektträger (VWR) wurden mit Ethylacetat in einem Ultraschallbad für 45 Minuten gereinigt und mit deionisiertem Wasser gewaschen. Auf eine Fläche von 25 x 25 mm² der Glasobjektträger wurden 625 µl einer wässrigen Lösung von Chymosin einer Konzentration von 25 g/L getropft und getrocknet. Anschließend wurden je zwei Glasobjektträger mit adsorbiertem Chymosin in Halterungen fixiert, wobei Abstandshalter einer Dicke von 125 µm verwendet wurden, um verschiedene Abstände von 125 µm, 250 µm, 375 µm oder 500 µm zwischen den jeweiligen Glasobjektträgern festzulegen. Nach der Fixierung wurden die Abstandshalter entfernt. Anschließend wurden die beiden Glasobjektträger in 200 ml einer Caseindispersion mit Konzentrationen von 1 g/L, 5 g/L, 10 g/L oder 20 g/L bei 40°C für 80 Minuten eingetaucht. Nach der Spaltungsreaktion wurden die zusammenhaftenden Glasobjektträger mit deionisiertem Wasser gewaschen und getrocknet.

Eine Haftung wurde als erfolgreich gewertet wenn die Glasobjektträger sowohl nach der Entfernung der Halterungen wie auch nach manueller Anwendung von leichtem mechanischem Stress zusammenhielten.

Die folgende Tabelle 2 zeigt die Ergebnisse der Haftung für die verschiedenen Abstände und Caseinkonzentrationen. Die angegebenen Ergebnisse sind jeweils Durchschnittswerte aus drei Einzelmessungen.

**Tabelle 2: Ergebnisse der Adhäsionsversuche**

| Entfernung [µm] | Caseinkonzentration [g/L] | Chymosinkonzentration [g/L] | pH-Wert | Ergebnis |
|---|---|---|---|---|
| 125 | 20 | 25 | 3 | + |
| 250 | 20 | 25 | 3 | + |
| 375 | 20 | 25 | 3 | + |
| 500 | 20 | 25 | 3 | - |
| 250 | 20 | 25 | 3 | + |
| 250 | 10 | 25 | 3 | + |
| 250 | 5 | 25 | 3 | +/- |
| 250 | 1 | 25 | 3 | - |
| 250 | 20 | kein Enzym | 3 | - |
| 250 | 20 | nur NaCl | 3 | - |
| 250 | 20 | 25 | 12 | - |

Der Tabelle 2 ist zu entnehmen, dass ein erfolgreiches Zusammenkleben der beiden Glasobjektträger bis zu einem Abstand von 375µm festgestellt werden konnte. Proben mit einem Abstand von 500 µm klebten nicht mehr zusammen. Hier wurde optisch bei großen Abständen ein Ablösen des präzipitierten Caseins vom Untergrund festgestellt. Feuchtes präzipitiertes Casein ist immer noch sehr weich und flexibel. Es wird daher angenommen, dass sich das Material durch die Schwerkraft vom Untergrund ablöst, wenn die aufgebrachte Schicht zu schwer wird. Wenn zwei Schichten jedoch zusammengebracht werden bevor eine Ablösung einer Schicht erfolgt, war der Vorgang des Klebens erfolgreich. Wird die Entfernung zu hoch, können die Schichten nicht aufeinandertreffen und ein Verkleben wird nicht ermöglicht.

Weiterhin wurde die Konzentration der Caseinlösung als Parameter untersucht. Es wurde festgestellt, dass Konzentrationen von 20 g/L und 10 g/L die Objektträger jeweils verklebten.

Eine Konzentration von 5 g/L zeigte variable Resultate und wurde als eine Art von Grenzbereich angesehen. Geringere Konzentrationen von 1 g/L zeigten kein Zusammenkleben der Objektträger mehr. In den erfolgreichen Versuchen wurde ein Zusammenkleben lediglich in den unteren Bereichen der Objektträger beobachtet, die mit Enzym funktionalisiert waren. Diese Beobachtung unterstützt die Annahme der enzymatischen Reaktion als treibende Kraft des Prozesses. Blindproben wurden durchgeführt, um die Notwendigkeit der enzymatischen Reaktion für das Verkleben zu belegen. Wenn die Experimente ohne Enzym durchgeführt wurden, wurde kein Verkleben der Objektträger beobachtet. Ebenso fand kein Verkleben statt, wenn das Enzym bei einem pH-Wert von 12 deaktiviert wurde. Um eine Abscheidung von Casein aufgrund von Salzeffekten auszuschließen, wurde als weitere Kontrollprobe eine Natriumchloridlösung anstelle der Enzymlösung auf den Objektträgern getrocknet, wobei ebenfalls kein Verkleben festgestellt wurde. Salzeffekte wurden als Kontrolle ausgeschlossen, weil das verwendete Chymosin mit NaCl formuliert war um dessen Langzeitstabilität zu erhöhen. Obwohl der Salzgehalt vor der Verwendung von 95% auf 33% reduziert wurde, war immer noch ein signifikanter Salzanteil vorhanden, welcher eine Proteinabscheidung induzieren kann. Es wurde jedoch festgestellt, dass eine solche Abscheidung für ein Verkleben nicht ausreicht. Insgesamt liefern die Kontrollen somit einen deutlichen Beleg, dass das Verkleben durch die enzymatische Reaktion ausgelöst wurde.

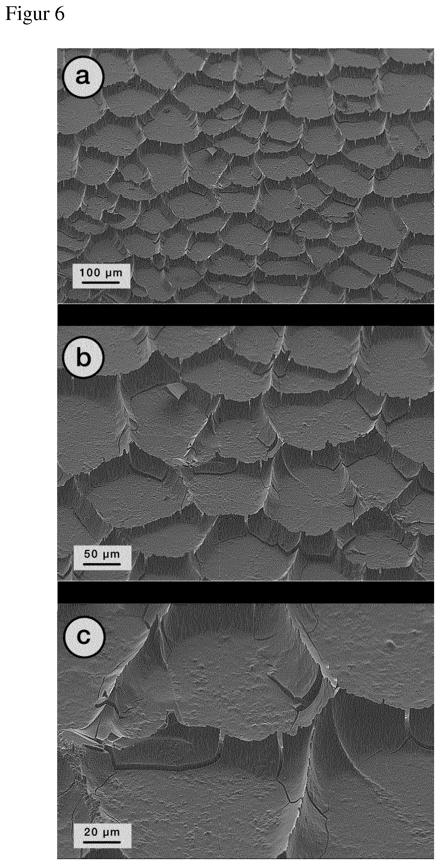

Weiterhin wurden die erhaltenen klebenden Caseinbeschichtungen nach einer Trennung der jeweiligen verklebten Objektträger mittels Elektronenmikroskopie (REM) unter Verwendung eines Zeiss "Neon 40" Rasterelektronenmikroskops untersucht. Aufnahmen der Proben wurden unter Verwendung des SE2-Detektors mit hohem topografischem Kontrast bei einer Beschleunigungsspannung von 2 kV aufgenommen. Die Figur 6 zeigt die REM-Aufnahme eines Glasobjektträgers nach dem Verkleben. Wie man in der Figuren 6a, b und c erkennen kann, wurde auf den Glasobjektträgern jeweils eine durchgehende Caseinschicht ausgebildet, die die gesamte Oberfläche des Trägers bedeckt. Weiter wurde auf der durchgehenden Beschichtung eine wabenartige Struktur ausgebildet, deren Ränder mit steigender Höhe dünner werden. Diese Struktur wurde jeweils auf beiden Objektträgern gefunden. Dieses legt nahe, dass das Casein auf beiden Seiten eine Beschichtung ausbildet, die durch ein komplexes Muster an Wänden verbunden sind. Es wird angenommen, dass eine solche Struktur ausgebildet wird, da sich zum einen eine massive Schicht aufgrund einer nicht ausreichenden Verfügbarkeit von Casein nicht ausbilden kann. Weiterhin betrug der Winkel von drei sich verzweigenden Wänden häufig etwa 120°, welches darauf hindeutet, dass die Ausbildung der wabenartigen Strukturen aufgrund der verschiedenen Polarität des hydrophoben gespaltenen Caseins und des hydrophilen Wassers erfolgt. Ferner härtet das Casein während des Trocknungsvorgangs aus und schrumpft, wodurch die Dicke der Seitenränder eine Funktion der Entfernung von dem jeweiligen Objektträger wird.

Dieses Beispiel zeigt, dass durch das erfindungsgemäße Verfahren Glasoberflächen haftend miteinander verklebt werden können. Ein Verkleben über Aufbringen von Chymosin und Abscheidung von Casein wurde unter den beschriebenen Bedingungen ebenfalls an Stahloberflächen wiederholt. Es wurde festgestellt, dass auch die Stahloberflächen miteinander verklebten. Dies zeigt, dass durch das erfindungsgemäße Verfahren ebenfalls Metalloberflächen haftend miteinander verklebt werden können.

Durch Aufbringen des Enzyms Chymosin gefolgt von der Abscheidung des Spaltungsproduktes des verwendeten Biopolymers wurde zwischen zwei Objekten eine diese verbindende Caseinschicht ausgebildet. Dies ermöglicht Anwendungen für Biomaterialien beispielsweise für medizinische Anwendungen wie medizinische Implantate, oder als biologisch abbaubarere Klebstoffe.

## Patentansprüche

1. Verfahren zur Beschichtung von Oberflächen durch enzymatische Reaktion von Casein, **dadurch gekennzeichnet, dass** das Verfahren die folgenden Schritte umfasst:
a) Aufbringen einer Protease ausgewählt aus der Gruppe umfassend Chymosin und/oder Pepsin auf die Oberfläche eines Substrats, und
b) In-Kontakt-Bringen der Protease mit dem abzuscheidenden Casein, wobei die Protease das Casein spaltet, wobei durch die Spaltung wenigstens zwei Spaltungsprodukte des Caseins mit unterschiedlicher Löslichkeit in einem Lösungsmittel entstehen, und wenigstens ein Spaltungsprodukt des Caseins mit geringerer Löslichkeit auf der Oberfläche des Substrats abgeschieden wird.

2. Verfahren nach Anspruch 1, **dadurch gekennzeichnet, dass** man die Protease mittels physikalischer Adsorption, oder ionischer, koordinativer oder kovalenter Bindung auf die Oberfläche aufbringt, wobei die kovalente Bindung an die Oberfläche über einen polymeren Abstandshalter vorzugsweise ausgewählt aus der Gruppe umfassend Polyethylenglycol, Polyvinylalkohol, Polyester und/oder Dextrane erfolgen kann.

3. Verfahren nach Anspruch 1 oder 2, **dadurch gekennzeichnet, dass** man die Protease ganz- oder teilflächig auf die Oberfläche aufbringt.

4. Verfahren nach einem der vorherigen Ansprüche, **dadurch gekennzeichnet, dass**
- das Casein in wässriger Lösung vorliegt, wobei die Konzentration des Caseins in der Lösung bevorzugt im Bereich von ≥ 0,01 g/L bis ≤ 50 g/L, vorzugsweise im Bereich von ≥ 0,1 g/L bis ≤ 10 g/L liegt, und/oder
- die Reaktionszeit zwischen Casein und Protease im Bereich von ≥ 1 min bis ≤ 240 min, vorzugsweise im Bereich von ≥ 5 min bis ≤ 60 min liegt, und/oder
- die Temperatur der Abscheidungsreaktion im Bereich von ≥ 0 °C bis ≤ 50 °C, vorzugsweise im Bereich von ≥ 30 °C bis ≤ 40 °C, liegt.

5. Verfahren nach einem der vorherigen Ansprüche, **dadurch gekennzeichnet, dass** man eine Beschichtung mit einer Schichtdicke im Bereich von ≥ 10 nm bis ≤ 50 µm, vorzugsweise im Bereich von ≥ 20 nm bis ≤ 1 µm, aufbringt.

6. Beschichteter Gegenstand, erhältlich durch das Verfahren nach einem der vorherigen Ansprüche, insbesondere ein medizinisches Implantat, ein biologisch abbaubareres Material, ein essbares Material, ein kolloidales Partikel oder eine zu verklebende Fläche, **dadurch gekennzeichnet, dass** der Gegenstand eine Casein-Beschichtung ausgebildet aus Casein-Micellen umfasst, wobei ein hydrophiler Teil des κ-Casein an der Stelle der Phenylalanin¹⁰⁵-Methionin¹⁰⁶-Bindung abgespalten wurde.

## Claims

1. A method for coating surfaces by enzymatic reaction of casein, **characterized in that** the method comprises the following steps:
a) applying a protease selected from the group comprising chymosin and/or pepsin to the surface of a substrate, and
b) contacting the protease with the casein to be deposited, the protease cleaving the casein, the cleavage giving rise to at least two cleavage products of the casein having differing solubility in a solvent, and at least one cleavage product of the casein having lower solubility being deposited on the surface of the substrate.

2. The method according to claim 1, **characterized in that** the protease is applied to the surface by means of physical adsorption, or ionic, coordinate or covalent bonding, it being possible to effect the covalent bonding to the surface via a polymeric spacer preferably selected from the group comprising polyethylene glycol, polyvinyl alcohol, polyesters and/or dextrans.

3. The method according to claim 1 or 2, **characterized in that** the protease is applied to the surface in a full-area or partial manner.

4. The method according to any of the preceding claims, **characterized in that**
- the casein is present in aqueous solution, the concentration of the casein in the solution being by preference within the range from ≥ 0.01 g/L to ≤ 50 g/L, preferably within the range from ≥ 0.1 g/L to ≤ 10 g/L, and/or
- the reaction time between casein and protease is within the range from ≥ 1 min to ≤ 240 min, preferably within the range from ≥ 5 min to ≤ 60 min, and/or
- the temperature of the deposition reaction is within the range from ≥ 0°C to ≤ 50°C, preferably within the range from ≥ 30°C to ≤ 40°C.

5. The method according to any of the preceding claims, **characterized in that** a coating having a layer thickness within the range from ≥ 10 nm to ≤ 50 µm, preferably within the range from ≥ 20 nm to ≤ 1 µm, is applied.

6. A coated article obtainable by means of the method according to any of the preceding claims, more particularly a medical implant, a biodegradable material, an edible material, a colloidal particle or a surface to be adhesively bonded, **characterized in that** the article comprises a casein coating formed of casein micelles, wherein a hydrophilic part of the κ-casein having been cleaved off at the site of the phenylalanine¹⁰⁵-methionine¹⁰⁶ bond.

## Revendications

1. Procédé de revêtement de surfaces par une réaction enzymatique de caséine, **caractérisé en ce que** le procédé comprend les étapes suivantes :
a) l'application d'une protéase choisie dans le groupe comprenant la chymosine et/ou la pepsine sur la surface d'un substrat, et
b) la mise en contact de la protéase avec la caséine à déposer, la protéase clivant la caséine, au moins deux produits de clivage de la caséine ayant différentes solubilités dans un solvant étant formés par le clivage, et au moins un produit de clivage de la caséine ayant une solubilité plus faible étant déposé sur la surface du substrat.

2. Procédé selon la revendication 1, **caractérisé en ce que** la protéase est appliquée sur la surface par adsorption physique, ou liaison ionique, coordinative ou covalente, la liaison covalente sur la surface pouvant avoir lieu par le biais d'un espaceur polymère, de préférence choisi dans le groupe comprenant le polyéthylène glycol, l'alcool polyvinylique, le polyester et/ou le dextrane.

3. Procédé selon la revendication 1 ou 2, **caractérisé en ce que** la protéase est appliquée sur l'ensemble ou sur une partie de la surface.

4. Procédé selon l'une quelconque des revendications précédentes, **caractérisé en ce que**
- la caséine se présente en solution aqueuse, la concentration de la caséine dans la solution se situant de préférence dans la plage allant de ≥ 0,01 g/l à ≤ 50 g/l, de préférence dans la plage allant de ≥ 0,1 g/l à ≤ 10 g/l, et/ou
- le temps de réaction entre la caséine et la protéase se situe dans la plage allant de ≥ 1 minute à ≤ 240 minutes, de préférence dans la plage allant de ≥ 5 minutes à ≤ 60 minutes, et/ou
- la température de la réaction de dépôt se situe dans la plage allant de ≥ 0 °C à ≤ 50 °C, de préférence dans la plage allant de ≥ 30 °C à ≤ 40 °C.

5. Procédé selon l'une quelconque des revendications précédentes, **caractérisé en ce qu'**un revêtement est appliqué en une épaisseur de couche dans la plage allant de ≥ 10 nm à ≤ 50 µm, de préférence dans la plage allant de ≥ 20 nm à ≤ 1 µm.

6. Article revêtu, pouvant être obtenu par le procédé selon l'une quelconque des revendications précédentes, notamment implant médical, matériau biodégradable, matériau comestible, particule colloïdale ou surface à coller, **caractérisé en ce que** l'article comprend un revêtement de caséine formé par des micelles de caséine, une partie hydrophile de la caséine κ ayant été clivée à l'emplacement de la liaison phénylalanine¹⁰⁵-méthionine¹⁰⁶.
